# FASCICULE DE BREVET EUROPEEN

(11) **EP 3 168 206 B1**
(45) Date de publication et mention de la délivrance du brevet: **27.11.2019**
(21) Numéro de dépôt: 16200315.6
(22) Date de dépôt: 13.01.2003
(51) Int. Cl.: C07C 59/29, C07C 59/46, C07C 69/67, C07C 69/73, C07C 31/135, C07C 33/12, A61P 17/00, A61K 8/34, A61K 8/44, A61Q 1/02, A61Q 19/00, A61Q 19/08, A61Q 5/00

(54) **COMPOSITIONS COMPRENANT DES DERIVES DE L'ACIDE JASMONIQUE, ET UTILISATION DE CES DERIVES POUR FAVORISER LA DESQUAMATION**
ZUSAMMENSETZUNGEN, DIE JASMONSÄURE-DERIVATE ENTHALTEN, UND EINSATZ DIESER DERIVATE ZUR BEGÜNSTIGUNG DER DESQUAMATION
COMPOSITIONS COMPRISING DERIVATIVES OF JASMONIC ACID, AND USE OF SAID DERIVATIVES TO PROMOTE EXFOLIATION

(30) Priorité: 04.02.2002 FR 0201279
(43) Date de publication de la demande: 17.05.2017
(62) Demande divisionnaire de: 03290081.3
(73) Titulaire: L'OREAL, 75008 Paris (FR)
(72) Inventeur: BOULLE, Christophe, 92110 CLICHY (FR); DALKO, Maria, 93601 AULNAY SOUS BOIS (FR); LEVEQUE, Jean-Luc, 75017 PARIS (FR); SIMONETTI, Lucie, 93601 AULNAY SOUS BOIS (FR)

(56) Documents cités:
- EP-A- 0 989 111
- WO-A-96/00206
- KIYOTA, H. ET AL: "Lipase-catalyzed preparation of both enantiomers of methyl jasmonate", TETRAHEDRON: ASYMMETRY, vol. 12, no. 7, 2001, pages 1035-1038, XP002214851,
- DATABASE CA [Online] CHEMICAL ABSTRACTS SERVICE, COLUMBUS, OHIO, US; TAKEUCHI, YASUTOMO ET AL: "Germination inducers containing cucurbic acids for parasitic plants", XP002214852, extrait de STN Database accession no. 131:28910 & JP 11 139907 A (INSTITUTE OF PHYSICAL AND CHEMICAL RESEARCH, JAPAN;NIPPON ZEON CO., LT) 25 mai 1999 (1999-05-25)
- SETO HIDEHARU ET AL: "Easy preparation of methyl 7-epi-jasmonate and four stereoisomers of methyl cucurbate, and assessment of the stereogenic effect of jasmonate o phytohormonal activities", CAPLUS, 1999, XP002214853,
- DATABASE CA [Online] CHEMICAL ABSTRACTS SERVICE, COLUMBUS, OHIO, US; BLECHERT, S. ET AL: "Structure-activity analyses reveal the existence of two separate groups o active octadecanoids in elicitation of the tendril-coiling response of Bryonia dioica", XP002214854, extrait de STN Database accession no. 130:279324
- DATABASE CA [Online] CHEMICAL ABSTRACTS SERVICE, COLUMBUS, OHIO, US; SETO, HIDEHARU ET AL: "Structure-activity relationships of (.+-.)-cucurbic acid analogs on the root growth of rice seedlings and height of young corn plants", XP002214855, extrait de STN Database accession no. 118:118844

## Description

La présente invention concerne des compositions cosmétiques ou pharmaceutiques comprenant des dérivés de l'acide jasmonique. L'invention concerne également des compositions, notamment cosmétiques pouvant être employées pour favoriser la desquamation de la peau et/ou stimuler le renouvellement épidermique et donc lutter contre le vieillissement cutané intrinsèque et/ou extrinsèque.

La desquamation est un phénomène naturel lié au fait que l'épiderme, qui constitue la couche supérieure de la peau, est en constante régénération. L'épiderme est constitué de plusieurs assises de cellules, dont la plus profonde est l'assise basale constituée de cellules indifférenciées. Au cours du temps, ces cellules vont différencier et migrer vers la surface de l'épiderme en constituant les différentes assises de celui-ci, jusqu'à former à la surface de l'épiderme les cornéocytes qui sont des cellules mortes qui s'éliminent par desquamation. Cette perte en surface est compensée par la migration de cellules de l'assise basale vers la surface de l'épiderme. Il s'agit du renouvellement perpétuel de la peau. Une élimination forcée de la couche cornée accélère le renouvellement et permet de lutter contre le vieillissement.

Dans le même temps ces cellules poursuivent leur différenciation dont le dernier stade est le cornéocyte. Il s'agit en fait de cellules mortes qui constituent la dernière couche de l'épiderme, c'est à dire la couche la plus externe encore appelée stratum corneum.

Le vieillissement cutané résultant de facteurs intrinsèques ou extrinsèques se traduit par l'apparition de rides et ridules, par le jaunissement de la peau qui développe un aspect parcheminé accompagné de l'apparition de taches pigmentaires, par la désorganisation des fibres d'élastine et de collagène entraînant une perte d'élasticité, de souplesse et de fermeté ou par l'apparition de télangiectasies. Certains de ces signes du vieillissement sont plus particulièrement liés au vieillissement intrinsèque ou physiologique, c'est-à-dire au vieillissement "normal" lié à l'âge ou chronobiologique, alors que d'autres sont plus spécifiques du vieillissement extrinsèque, c'est-à-dire du vieillissement provoqué d'une manière générale par l'environnement; il s'agit plus particulièrement du photovieillissement dû à l'exposition au soleil, à la lumière ou à tout autre rayonnement.

L'invention s'intéresse au vieillissement intrinsèque ou physiologique ainsi qu'au vieillissement extrinsèque.

Les changements de la peau dus au vieillissement intrinsèque sont la conséquence d'une sénescence génétiquement programmée où interviennent des facteurs endogènes. Ce vieillissement intrinsèque provoque notamment un ralentissement du renouvellement des cellules de la peau, ce qui se traduit essentiellement par l'apparition d'altérations cliniques telles que la réduction du tissu adipeux sous-cutané et l'apparition de fines rides ou ridules, et par des changements histopathologiques tels qu'une augmentation du nombre et de l'épaisseur des fibres élastiques, une perte de fibres verticales de la membrane du tissu élastique, et la présence de grands fibroblastes irréguliers dans les cellules de ce tissu élastique. Au contraire, le vieillissement extrinsèque entraîne des altérations cliniques telles que des rides épaisses et la formation d'une peau molle et tannée, et des changements histopathologiques tels qu'une excessive accumulation de matière élastique dans le derme supérieur et une dégénérescence des fibres de collagène. On connaît dans l'art antérieur divers agents destinés à lutter contre le vieillissement cutané.

Ainsi, le brevet US-A-4603146 décrit l'emploi d'acide rétinoïque et de ses dérivés dans des compositions cosmétiques, en vue de lutter contre le vieillissement cutané.

Par ailleurs, de nombreux brevets et publications (voir par exemple la demande EP-A-413528) ainsi que de nombreuses compositions cosmétiques du commerce enseignent l'emploi des α-hydroxyacides comme l'acide lactique, l'acide glycolique ou encore l'acide citrique pour traiter le vieillissement cutané.

On connaît enfin les β-hydroxyacides et plus spécialement l'acide salicylique ainsi que ses dérivés pour leur propriétés desquamantes (voir les documents WO-A-93/10756 et US-A-4 767 750).

JP2001207188 décrit le composé dihydrojasmonate de méthyle comme accélérateur de l'activité de la péroxydase et suppresseur de la production de lipides péroxydés dans des compositions parfumantes. Tous ces composés ont une action contre le vieillissement de la peau en favorisant la desquamation, c'est-à-dire l'élimination des cellules mortes situées à la surface de la couche cornée de l'épiderme. Cette propriété "desquamante" est aussi appelée, souvent à tort, propriété kératolytique.

Mais les composés de l'art antérieur présentent également des effets secondaires, qui consistent en des picotements, des tiraillements, des échauffements et des rougeurs désagréables pour l'utilisateur.

On constate donc que subsiste le besoin d'agents antivieillissement ayant une action au moins aussi efficace que celle des composés de l'art antérieur, mais ne présentant pas leurs inconvénients.

L'invention a pour but de pallier ces inconvénients de l'art antérieur et de proposer de nouveaux composés susceptibles de favoriser la desquamation de la peau et/ou de stimuler le renouvellement épidermique, dont l'utilisation n'entraînerait pas de picotements, de tiraillements, d'échauffements ou de rougeurs désagréables pour l'utilisateur.

Ainsi, l'invention a pour objet une composition cosmétique selon la revendication 1.

On a constaté que les composés selon l'invention présentent une bonne solubilité dans l'eau, ce qui peut faciliter leur mise en œuvre.

Les composés selon l'invention répondent à la formule (I) suivante : 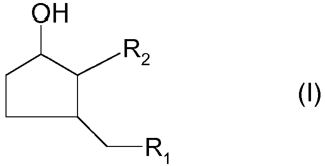 tel que définis dans la revendication 1.

De préférence, le radical R₁ est choisi parmi -COOR', -CONR'R" et -CH₂OR' avec R' et R" désignant, indépendamment l'un de l'autre, un atome d'hydrogène ou un radical hydrocarboné, linéaire, ramifié ou cyclique, saturé ou insaturé, ayant 1 à 18 atomes de carbone, notamment 1 à 12 atomes de carbone, et en particulier 1 à 8 atomes de carbone.

Plus particulièrement, R₁ est choisi parmi les radicaux -COOH, -CH₂OH,-COOCH₃, -COOC₂H₅, -COOC₃H₇, -CONHCH₃ et -CONHC₂H₅.

De préférence, le radical R₂ est un radical hydrocarboné, linéaire, ramifié ou cyclique, saturé ou insaturé, ayant 1 à 18 atomes de carbone, notamment 1 à 12 atomes de carbone, et en particulier 1 à 8 atomes de carbone.

Plus particulièrement, R₂ représente un radical hydrocarboné linéaire, saturé ou comportant une insaturation, ayant 2 à 6 atomes de carbone, et notamment un radical -CH₂-CH=CH-C₂H₅ ou un radical -(CH₂)₄-CH₃.

Les sels des composés utilisables selon l'invention sont en particulier choisis parmi les sels de métal alcalin ou alcalino-terreux, ou encore parmi les sels de zinc, de magnésium ou de strontium, d'une amine organique ou les sels d'ammonium quaternaires.

Les sels des composés conformes à l'invention sont en particulier choisis parmi les sels d'un acide minéral ou organique notamment les chlorhydrates, bromhydrates ou citrates.

Parmi les composés susceptibles d'être employés dans le cadre de l'invention, on peut citer :
- l'acide 3-hydroxy-2-[(2Z)-2-pentenyl]-cyclopentaneacétique,
- l'acide 3-hydroxy-2-pentyl-cyclopentaneacétique,

La quantité de composé de formule (I) utilisable selon l'invention dépend bien évidemment de l'effet recherché et doit être en une quantité efficace pour favoriser la desquamation de la peau et/ou stimuler le renouvellement épidermique et donc lutter contre le vieillissement cutané intrinsèque et/ou extrinsèque.

A titre d'exemple, la quantité de composé de formule (I) utilisable selon l'invention peut aller par exemple de 0,01 à 20% et de préférence de 0,5 à 10%, et notamment de 1 à 5% en poids, par rapport au poids total de la composition.

La composition comprenant les composés selon l'invention, seuls ou en mélange, peut comprendre par ailleurs un milieu physiologiquement acceptable, c'est-à-dire un milieu compatible avec toutes les matières kératiniques telles que la peau, le La composition comprenant les composés selon l'invention, seuls ou en mélange, peut comprendre par ailleurs un milieu physiologiquement acceptable, c'est-à-dire un milieu compatible avec toutes les matières kératiniques telles que la peau, le cuir chevelu, les ongles, les muqueuses, les yeux et les cheveux ou toute autre zone cutanée du corps. Cette composition peut être une composition cosmétique ou pharmaceutique et peut donc comprendre un milieu cosmétiquement ou pharmaceutiquement acceptable.

Le milieu physiologiquement acceptable peut comprendre de l'eau, des solvants organiques tels qu'un alcool en C₁-C₈, notamment l'éthanol, l'isopropanol, le tert-butanol, le n-butanol; un polyol tel que la glycérine ; un glycol comme le butylène glycol, l'isoprène glycol, le propylène glycol, les polyéthylène glycols tels que le PEG-8 ; les éthers de polyol.

La composition peut également comprendre une phase grasse, qui peut comprendre des huiles, des gommes, des cires usuellement utilisées dans le domaine d'application considéré. Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Lorsque la composition est une émulsion, la proportion de la phase grasse peut aller de 5% à 80% en poids, et de préférence de 5% à 50% en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3% à 30% en poids, et de préférence de 0,5 à 20% en poids par rapport au poids total de la composition. L'émulsion peut, en outre, contenir des vésicules lipidiques.

Lorsque la composition est une solution ou un gel huileux, la phase grasse peut représenter plus de 90% du poids total de la composition.

La composition peut contenir également des adjuvants habituels dans le domaine considéré, tels que les tensioactifs, les émulsionnants, les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes, les actifs cosmétiques ou pharmaceutiques. Les quantités de ces différents adjuvants sont celles classiquement utilisées dans le domaine cosmétique, et par exemple de 0,01% à 10% du poids total de la composition. Ces adjuvants, selon leur nature, peuvent être introduits dans la phase grasse, dans la phase aqueuse et/ou dans les sphérules lipidiques.

Comme tensioactifs susceptibles d'être utilisés, on peut citer par exemple le stéarate de glycérol, le polysorbate 60 et le mélange de PEG-6/PEG-32/Glycol Stéarate vendu sous la dénomination de TefoseR 63 par la société Gattefosse.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

Parmi les actifs hydrophiles, on peut citer les protéines ou les hydrolysats de protéine, les acides aminés, les polyols, l'urée, l'allantoïne, les sucres et les dérivés de sucre, les vitamines hydrosolubles, les extraits végétaux et les hydroxyacides. Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention la composition peut associer au moins un composé de formule (I) à d'autres agents actifs, tels que :
- les agents améliorant la repousse et/ou sur le ralentissement de la chute des cheveux, comme par exemple les esters d'acide nicotinique, dont notamment le nicotinate de tocophérol, le nicotinate de benzyle et les nicotinates d'alkyles en C1-C6 comme les nicotinates de méthyle ou d'hexyle, les dérivés de pyrimidine, comme le 2,4-diamino 6-piperidinopyrimidine 3-oxyde ou "Minoxidil", les agents favorisant la repousse des cheveux comme ceux décrits par la demanderesse dans la demande de brevet européen publiée sous le numéro 0648488 ;
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens comme par exemple l'ibuprofène et ses sels, le diclofénac et ses sels, l'acide acétylsalicylique, l'acétaminophène ou l'acide glycyrrhizique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides α- et β-hydroxycarboxyliques ou β-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'α-tocophérol ou ses esters, les superoxyde dismutases, certains chélatants de métaux ou l'acide ascorbique et ses esters ;
- les anti-séborrhéiques tels que la progestérone ;
- les antipelliculaires comme l'octopirox ou la pyrithione de zinc ;
- les antiacnéiques comme l'acide rétinoïque ou le peroxyde de benzoyle ;
- les extraits d'origine végétale, marine ou bactérienne.

La composition peut se présenter sous toutes les formes galéniques envisageables.

Notamment, la composition peut avoir la forme de solution aqueuse, alcoolique, hydroalcoolique ou huileuse; de dispersion du type lotion ou sérum; d'émulsion eau-dans-huile, huile-dans-eau ou multiple; de suspension; de microcapsules ou microparticules; de dispersions vésiculaires de type ionique et/ou non ionique; de lotion aqueuse, huileuse ou sous forme de sérum; de capsules, de granulés, de sirops, de comprimés; de mousse, de préparation solide; de composition pour aérosol comprenant également un agent propulseur sous pression.

La composition selon l'invention peut se présenter sous la forme d'une composition pour soins capillaires, notamment un shampooing, une lotion de mise en plis, une lotion traitante, une crème ou un gel coiffant, une composition de teinture, notamment d'oxydation, des lotions restructurantes pour les cheveux, une composition de permanente (notamment une composition pour le premier temps d'une permanente), une lotion ou un gel antichute, un shampooing antiparasitaire.

Elle peut également se présenter sous la forme d'une composition de nettoyage, de protection, de traitement ou de soin pour le visage, pour les mains, pour les pieds, pour les grands plis anatomiques ou pour le corps (par exemple crèmes de jour, crème de nuit, crème démaquillante, composition anti-solaire, lait corporels composition pour le bain; une composition désodorisante comprenant par exemple un agent bactéricide; une composition après-rasage; une composition épilatoire; une composition contre les piqûres d'insectes; une composition anti-douleur; une composition pour traiter certaines maladies de la peau comme l'eczéma, la rosacée, le psoriasis, les lichens, les prurits sévères.

La composition selon l'invention trouve une application toute particulière comme composition cosmétique ou pharmaceutique destinée au soin de la peau du corps, du visage et/ou du cuir chevelu, notamment pour favoriser la desquamation de la peau, stimuler le renouvellement épidermique, lutter contre les signes du vieillissement cutané, améliorer l'éclat du teint et/ou lisser la peau du visage.

L'invention est illustrée plus en détail dans les exemples suivants.

### Exemple 1 : synthèse de l'acide (1R,2R) 3-hydroxy-2-[(2Z)-2-pentényl]-cyclopentaneacétique (+/-) de formule :

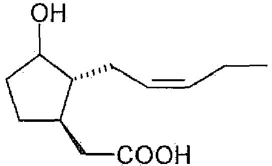

### 1/ étape 1 : synthèse de l'acide jasmonique (+/-) ou acide (1R,2R) 3-oxo-2-[(2Z)-2-pentenyl]-cyclopentaneacétique (+/-)

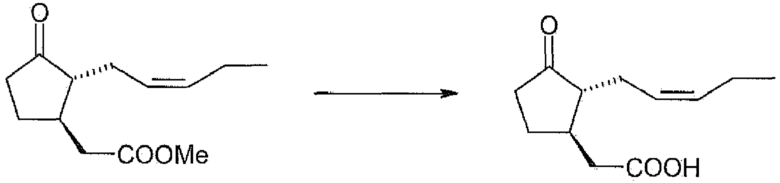

Dans un tricol de 250 ml muni d'un réfrigérant, d'un thermomètre et d'une agitation magnétique, on dissout 15 g (66,9 mmol) de (+/-) jasmonate de méthyle dans 150 ml d'acétone. On additionne lentement 10 ml de solution aqueuse de soude (5,35 g, 133,7 mmol). Le mélange est agité pendant 5 heures à température ambiante. L'acétone est alors évaporée sous vide puis la phase aqueuse résiduelle est lavée par de l'acétate d'éthyle (2 x 30 ml). La phase aqueuse est acidifiée par de l'acide chlorhydrique jusqu'à pH=2, puis extraite par du dichlorométhane (3 x 30 ml).

La phase organique est séchée sur sulfate de sodium, filtrée sur papier filtre puis concentrée. L'huile marron clair obtenue est séchée sous vide.

On obtient 13,6 g d'acide jasmonique (+/-) soit un rendement de 97%.

Le spectre RMN ¹H et le spectre de masse (ionisation négative) sont conformes à la structure attendue.

### 2/ étape 2 : synthèse de l'acide (1R,2R) 3-hydroxy-2-[(2Z)-2-pentenyl]-cyclopentaneacétique (+/-)

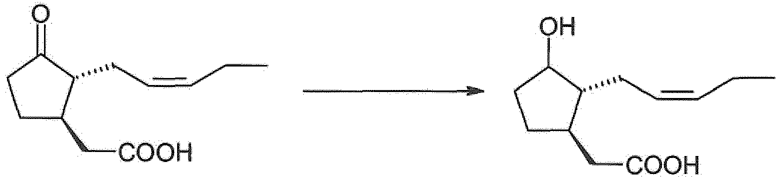

Dans un tricol de 50 ml muni d'un réfrigérant, d'un thermomètre et d'une agitation magnétique, on dissout 1 g (4,8 mmol) d'acide jasmonique (+/-) dans 15 ml d'éthanol absolu. On ajoute 430 mg (11,4 mmol) de borohydrure de sodium NaBH₄. Le mélange est agité pendant 4 heures à 50°C. Une fois la réaction terminée, on ajoute lentement 5 ml d'eau. Le précipité formé est filtré. Le filtrat est acidifié par de l'acide chlorhydrique jusqu'à pH=5 puis extrait par de l'acétate d'éthyle (3 x 30 ml). La phase organique est séchée sur sulfate de sodium, filtrée sur papier filtre puis concentrée. L'huile incolore obtenue est purifiée par chromatographie sur gel de silice (éluant : dichlorométhane/méthanol). L'huile incolore obtenue est séchée sous vide.

On obtient 400 mg du composé recherché, soit un rendement de 40%.

Le spectre RMN ¹H est conforme à la structure attendue.

### Exemple 3 : Synthèse de l'acide (1R,2R) 3-hydroxy-2-pentyl-cyclopentaneacétique (+/-) de formule :

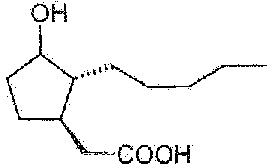

### 1/ étape 1 : synthèse de l'acide dihydrojasmonique (+/-) ou acide (1R,2R) 3-oxo-2-pentyl-cyclopentaneacétique (+/-) de formule :

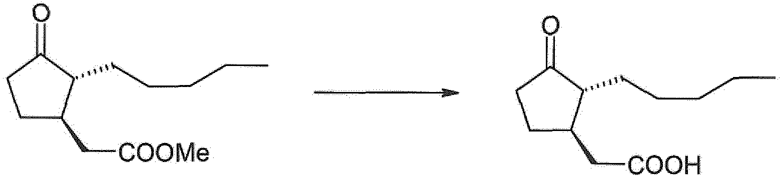

Dans un tricol de 250 ml muni d'un réfrigérant, d'un thermomètre et d'une agitation magnétique, on dissout 5 g (23,6 mmol) de (+/-) dihydrojasmonate de méthyle dans 50 ml d'acétone. On ajoute lentement 10 ml de solution aqueuse de soude (1,76 g, 44 mmol). Le mélange est agité pendant 5 heures à température ambiante. L'acétone est alors distillée sous vide puis la phase aqueuse résiduelle est lavée par de l'acétate d'éthyle (2 x 20 ml). La phase aqueuse est acidifiée par de l'acide chlorhydrique jusqu'à pH=2. Cette phase est alors extraite par du dichlorométhane (2 x 30 ml). La phase organique est séchée sur sulfate de sodium, filtrée sur papier filtre puis concentrée. L'huile obtenue est purifiée par chromatographie sur gel de silice (éluant : dichlorométhane / méthanol). L'huile obtenue est séchée sous vide.

On obtient 1,7 g du composé recherché, soit un rendement de 36%.

Le spectre RMN ¹H est conforme à la structure attendue.

### 2/ étape 2 : synthèse de l'acide (1R,2R) 3-hydroxy-2-pentyl-cyclopentane-acétique

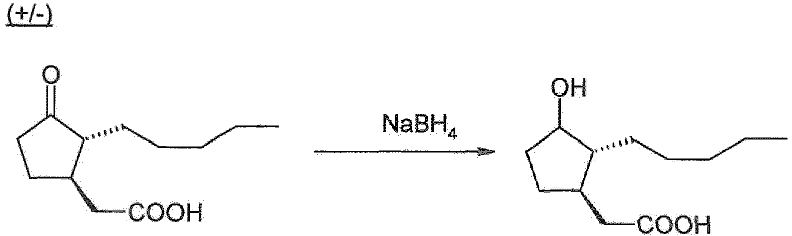

On prépare ce composé de manière similaire à celle décrite à l'étape 2 de l'exemple 1, en faisant réagir de l'acide dihydrojasmonique avec du borohydrure de sodium NaBH₄, dans l'éthanol, pendant 4 heures à 50°C.

On obtient le composé recherché avec un rendement de 45%.

Le spectre RMN ¹H est conforme à la structure attendue.

### Exemple 5 : tests d'activité

On étudie le pouvoir kératolytique de plusieurs composés selon l'invention. Ce test consiste en un comptage de cornéocytes libérés après incubation des lots de stratum corneum isolé en présence des composés testés.

On utilise du stratum corneum isolé par trypsine/chaleur à partir de plasties de chirurgie. On utilise plusieurs échantillons de stratum corneum différents. Des On utilise du stratum corneum isolé par trypsine/chaleur à partir de plasties de chirurgie. On utilise plusieurs échantillons de stratum corneum différents. Des disques de 4 mm de diamètre sont coupés à l'emporte pièce et disposés au fond d'une boite de 96 puits.

### Test 1

On prépare une solution à 1% en poids de composé de l'exemple 3 dans un tampon PBS supplémenté à 0,1% en Triton X100. Le pH de la solution est réajusté à 7,4.

On ajoute dans chaque puits 50 microlitres de solution à tester ou de solution témoin (tampon PBS supplémenté à 0,1% en TritonX100). On laisse incuber à 37°C sous agitation pendant 24 heures.

On prélève alors 10 microlitres de solution, que l'on dispose en cellule de Mallassez. Les cornéocytes libérés sont comptés sous microscope.

On obtient les résultats suivants, exprimés en nombre de cornéocytes libérés par microlitre, moyennés pour trois essais. Les fragments de cornéocytes ne sont pas comptés.

| | Echantillon 1 * | Echantillon 2 * | Echantillon 3 * | Moyenne |
|---|---|---|---|---|
| Exemple 3 | 28 ± 9 | 56 ± 7 | 101 ± 11 | 61 ± 33 |
| Témoin | 4 ± 4 | 8 ± 3 | 17 ± 8 | 9 ± 8 |

| | | | | |
|---|---|---|---|---|
| * moyenne sur trois essais | | | | |

Le nombre de cornéocytes libérés après incubation du stratum corneum isolé avec le composé selon l'invention est très supérieur au nombre libéré en présence du tampon seul.

### Test 2

On prépare une solution à 1% en poids de composé de l'exemple 1 ou d'acide jasmonique (comparatif) dans un tampon PBS supplémenté à 0,1% en Triton X100. Le pH des solutions est réajusté à 7,4.

On ajoute dans chaque puits 50 microlitres de solution à tester ou de solution témoin (tampon PBS supplémenté à 0,1% en TritonX100). On laisse incuber à 37°C sous agitation pendant 24 heures.

On prélève alors 10 microlitres de solution, que l'on dispose en cellule de Mallassez. Les cornéocytes libérés sont comptés sous microscope.

On obtient les résultats suivants, exprimés en nombre de cornéocytes libérés par microlitre, moyennés pour trois essais. Les fragments de cornéocytes ne sont pas comptés.

| | Moyenne (3 essais par échantillon, 3 échantillons différents) |
|---|---|
| Exemple 1 | 26 ± 7 |
| Acide jasmonique | 15 ± 5 |
| Témoin | 9 ± 3 |

Les résultats nous indiquent que le composé de l'exemple 1 permet une meilleure libération de cornéocytes que l'acide jasmonique par rapport au témoin.

### Test 3

On prépare une solution à 2% en poids de composé de l'exemple 3, ou d'acide 2-hydroxy-4-octanoyl-benzoïque (comparatif), dans une solution aqueuse comprenant 50% en poids de polyéthylène glycol (PEG 8) et 30% en poids d'éthanol.

On ajoute dans chaque puits 50 microlitres de solution à tester ou de solution témoin. On laisse incuber à 37°C sous agitation pendant 24 heures.

On prélève alors 10 microlitres de solution, que l'on dispose en cellule de Mallassez. Les cornéocytes libérés sont comptés sous microscope.

On obtient les résultats suivants, exprimés en nombre de cornéocytes libérés par microlitre, moyennés pour trois essais. Les fragments de cornéocytes ne sont pas comptés.

| | Echantillon 1 * | Echantillon 2 * | Moyenne |
|---|---|---|---|
| Exemple 3 | 2 ± 3 | 3 ± 1 | 2,5 ± 2 |
| Acide 2-hydroxy-4-octanoyl-benzoïque | 3 ± 3 | 4 ± 3 | 3,5 ± 3 |
| Témoin | 1 ± 1 | 1 ± 1 | 1 ± 1 |

| | | | |
|---|---|---|---|
| * moyenne sur trois essais | | | |

### Exemple 6

On prépare une émulsion comprenant (% en poids) :

| | |
|---|---|
| - composé de l'exemple 1 | 1% |
| - isostéarate de propylène glycol | 13% |
| - polyéthylène glycol (8 OE) | 5% |
| - propylène glycol | 3% |
| - pentylène glycol | 3% |
| - stéarate de glycéryle et stéarate de polyéthylène glycol (100 OE) | 5% |
| - mono-stéarate de sorbitane oxyéthyléné (20 OE) | 0,5% |
| - alcool cétylique oxyéthyléné (20 OE) oxypropyléné (5 OP) | 1% |
| - gélifiant | 0,5% |
| - benzoates d'alkyle en C₁₂₋₁₅ | 4% |
| - éthanol | 3% |
| - hydroxyde de sodium | 0,12% |
| - conservateurs | qs |
| - eau | qsp 100% |

## Revendications

1. Composition cosmétique comprenant, dans un milieu cosmétiquement acceptable, au moins un composé de formule (I) : 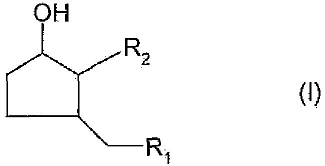 dans laquelle :
- R₁ est un radical -COOH ;
- R₂ est un radical hydrocarboné, linéaire, ramifié ou cyclique, saturé ou insaturé, ayant 1 à 18 atomes de carbone ;
et ses sels correspondants ;
et un adjuvant cosmétique choisi parmi un polyol, les cires, les gélifiants hydrophiles ou lipophiles, les antioxydants, les charges, les filtres, les absorbeurs d'odeur, les matières colorantes, les huiles minérales, les huiles végétales, les huiles animales , les huiles siliconées, les huiles fluorées ;
les actifs hydrophiles choisis parmi les protéines ou les hydrolysats de protéine, les acides aminés, l'urée, l'allantoïne, les sucres et les dérivés de sucre, les vitamines hydrosolubles, les extraits végétaux et les hydroxyacides ;
les actifs lipophiles choisis parmi le rétinol et ses dérivés, le tocophérol et ses dérivés, les acides gras essentiels, les céramides, les huiles essentielles, l'acide salicylique et ses dérivés.

2. Composition selon la revendication précédente, dans laquelle le radical R₂ est un radical hydrocarboné, linéaire, ramifié ou cyclique, saturé ou insaturé, ayant 1 à 12 atomes de carbone, et en particulier 1 à 8 atomes de carbone.

3. Composition selon l'une des revendications précédentes, dans laquelle le radical R₂ représente un radical hydrocarboné linéaire, saturé ou comportant une insaturation, ayant 2 à 6 atomes de carbone, et notamment un radical -CH₂-CH=CH-C₂H₅ ou un radical -(CH₂)₄-CH₃.

4. Composition selon l'une des revendications précédentes, dans laquelle le composé de formule (I) est choisi parmi :
- l'acide 3-hydroxy-2-[(2Z)-2-pentenyl]-cyclopentaneacétique,
- l'acide 3-hydroxy-2-pentyl-cyclopentaneacétique.

5. Composition selon l'une des revendications précédentes, dans laquelle le composé de formule (I) est l'acide 3-hydroxy-2-pentyl-cyclopentaneacétique.

6. Composition selon l'une des revendications précédentes, dans laquelle le sel du composé de formule (I) est choisi parmi les sels de métal alcalin ou alcalino-terreux, les sels de zinc, de magnésium ou de strontium, les sels d'une amine organique ou les sels d'ammonium quaternaires.

7. Composition selon l'une des revendications précédentes, dans laquelle le sel du composé de formule (I) est choisi parmi les sels de métal alcalin.

8. Composition selon l'une des revendications précédentes, dans laquelle le composé (I) est présent en une quantité de 0,01 à 20%, de préférence de 0,5 à 10%, et notamment de 1 à 5% en poids, par rapport au poids total de la composition.

## Patentansprüche

1. Kosmetische Zusammensetzung, umfassend in einem kosmetisch unbedenklichen Medium mindestens eine Verbindung der Formel (I): 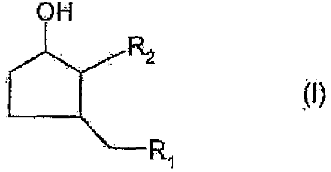 in der:
- R₁ für einen -COOH-Rest steht;
- R₂ für einen gesättigten oder ungesättigten, linearen, verzweigten oder cyclischen Kohlenwasserstoffrest mit 1 bis 18 Kohlenstoffatomen steht;
und die entsprechenden Salze davon;
und ein kosmetisches Adjuvans, ausgewählt aus einem Polyol, Wachsen, hydrophilen oder lipophilen Geliermitteln, Antioxidationsmitteln, Füllstoffen, Filtern, geruchsabsorbierenden Substanzen, Farbstoffen, Mineralölen, Pflanzenölen, tierischen Ölen, Silikonölen und Fluorölen;
hydrophile Wirkstoffe ausgewählt aus Proteinen bzw. Proteinhydrolysaten, Aminosäuren, Harnstoff, Alantoin, Zuckern und zuckerderivaten, wasserlöslichen Vitaminen, Pflanzenextrakten und Hydroxysäuren;
lipophile Wirkstoffe, ausgewählt aus Retinol und dessen Derivaten, Tocopherol und dessen Derivaten, essenziellen Fettsäuren, Ceramiden, essenziellen Ölen, Salicylsäure und deren Derivaten.

2. Zusammensetzung nach dem vorhergehenden Anspruch, wobei der Rest R₂ für einen gesättigten oder ungesättigten, linearen, verzweigten oder cyclischen Kohlenwasserstoffrest mit 1 bis 12 Kohlenstoffatomen und insbesondere 1 bis 8 Kohlenstoffatomen steht.

3. Zusammensetzung nach einem der vorhergehenden Ansprüche, wobei der Rest R₂ für einen gesättigten oder eine Ungesättigtheit enthaltenden, linearen Kohlenwasserstoffrest mit 2 bis 6 Kohlenstoffatomen und insbesondere einen -CH₂-CH=CH-C₂H₅-Rest oder einen -(CH₂)₄-CH₃-Rest steht.

4. Zusammensetzung nach einem der vorhergehenden Ansprüche, wobei die Verbindung der Formel (I) aus
- 3-Hydroxy-2-[(2Z)-2-pentenyl]cyclopentanessigsäure und
- 3-Hydroxy-2-pentylcyclopentanessigsäure ausgewählt ist.

5. Zusammensetzung nach einem der vorhergehenden Ansprüche, wobei sich bei der Verbindung der Förmel (I) um 3-Hydroxy-2-pentylcyclopentanessigsäure handelt.

6. Zusammensetzung nach einem, der vorhergehenden Ansprüche, wobei das Salz der Verbindung der Formel (I) aus Alkali- oder Erdalkalimetallsalzen, Zink-, Magnesium- oder Strontiumsalzen, Salzen eines organischen Amins oder quaternären Ammoniumsalzen ausgewählt ist.

7. Zusammensetzung nach einem der vorhergehenden Ansprüche, wobei das Salz der Verbindung der Formel (I) aus Alkalimetallsalzen ausgewählt ist.

8. Zusammensetzung nach einem der vorhergehenden Ansprüche, wobei die Verbindung (I) in einer Menge von 0,01 bis 20 Gew.-%, vorzugsweise 0,5 bis 10 Gew.-% und insbesondere 1 bis 5 Gew.-%, bezogen auf das Gesamtgewicht der Zusammensetzung, vorliegt.

## Claims

1. Cosmetic composition comprising, in a cosmetically acceptable medium, at least one compound of formula (I) : 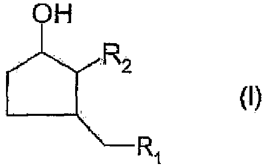 in which:
- R₁ is a -COOH radical;
- R₂ is a linear, branched or cyclic, saturated or unsaturated hydrocarbon-based radical containing 1 to 18 carbon atoms;
and the corresponding salts thereof;
and a cosmetic adjuvant chosen from a polyol, waxes, hydrophilic or lipophilic gelling agents, antioxidants, fillers, filters, odour absorbers, dyestuffs, mineral oils, plant oils, animal oils, silicone oils and fluoro oils;
hydrophilic active agents chosen from proteins or protein hydrolysates, amino acids, urea, allantoin, sugars and sugar derivatives, water-soluble vitamins, plant extracts and hydroxy acids;
lipophilic active agents chosen from retinol and derivatives thereof, tocopherol and derivatives thereof, essential fatty acids, ceramides, essential oils, arid salicylic acid and derivatives thereof.

2. Composition according to the preceding claim, in which the radical R₂ is a linear, branched or cyclic, saturated or unsaturated hydrocarbon-based radical containing 1 to 12 carbon atoms, and in particular 1 to 8 carbon atoms.

3. Composition according to either of the preceding claims, in which the radical R₂ represents a linear hydrocarbon-based radical, which is saturated or comprising an unsaturation, containing 2 to 6 carbon atoms, and especially a -CH₂-CH=CH-C₂H₅ radical or a -(CH₂)₄-CH₃ radical.

4. Composition according to one of the preceding claims, in which the compound of formula (I) is chosen from:
- 3-hydroxy-2-[(2z)-2-pentenyl]cyclopentaneacetic acid,
- 3-hydroxy-2-pentylcyclopentaneacetic acid.

5. Composition according to one of the preceding claims, in which the compound of formula (I) is 3-hydroxy-2-pentylcyclopenta-neacetic acid.

6. Composition according to one of the preceding claims, in which the salt of the compound of formula (I) is chosen from alkali metal or alkaline-earth metal salts, zinc, magnesium or strontium salts, salts of an organic amine or quaternary ammonium salts.

7. Composition according to one of the preceding claims, in which the salt of the compound of formula (I) is chosen from the alkali metal salts.

8. Composition according to one of the preceding claims, in which the compound (I) is present in an amount from 0.01% to 20%, preferably from 0.5% to 10% and especially from 1% to 5% by weight relative to the total weight of the composition.
